# EUROPEAN PATENT APPLICATION

(11) **EP 3 175 772 A1**
(43) Date of publication of application: **07.06.2017**
(21) Application number: 16827472.8
(22) Date of filing: 31.03.2016
(51) Int. Cl.: A61B 1/00

(54) **ENDOSCOPE**

(30) Priority: 21.07.2015 JP 2015144144
(71) Applicant: Olympus Corporation, Tokyo 192-8507 (JP)
(72) Inventor: ARAI, Keiichi, Hachioji-shi Tokyo 192-8507 (JP)
(74) Representative: Schicker, Silvia
(86) International application number: PCT/JP2016/060686
(87) International publication number: WO 2017/013908

(57) **Abstract**

An endoscope 1 includes a bending section 3 provided in an insertion section 2 inserted into a subject, the bending section 3 being bendable, and a switching section 20 capable of changing rigidity on a distal end side of the bending section 3.

## Description

### Technical Field

The present invention relates to an endoscope having a characteristic in a bending section provided in an insertion section.

### Background Art

In a medical field, an endoscope has been used with which observation and various kinds of curative treatment can be performed by inserting an elongated insertion section into a body. In the endoscope, an observation optical system for picking up an observation image of an observation region is provided at a distal end portion of the insertion section.

A bending section is provided on a distal end side of the insertion section of the endoscope. The bending section is configured to bend in upward and downward two directions or upward, downward, left, and right four directions by, for example, turnably concatenating a plurality of bending pieces.

The bending section performs a bending motion, for example, according to operation of a rotation knob, which is a bending section operation device, provided in an operation section. With the endoscope including the bending section, it is possible to easily perform insertion into a deep part in the body and direct the observation optical system of the distal end portion to a desired direction.

In an endoscope in which a bending radius of a bending section is reduced, the bending section is configured to bend 180° or more. Consequently, it is possible to observe an opposite direction of an insertion direction of an insertion section in a stomach or in a large intestine, for example, that is, perform backward observation (also referred to as reverse observation).

In this way, with the endoscope including the bending section in recent years, it is possible to photograph an observation region from a front and obtain a satisfactory observation image.

However, in the endoscope in which the bending radius of the bending section is reduced, a curvature of the bending section in a bent state increases. As a result, it is difficult for a treatment instrument to smoothly pass in a treatment instrument channel in the bending section.

In addition, distal end rigid length is short in the endoscope in which the bending radius of the bending section is reduced. Therefore, depending on a position of a lesion part, when endoscopic submucosal dissection (hereinafter described as ESD) is performed, it is difficult to dispose, for example, an IT knife (hereinafter abbreviated as knife), which is a treatment instrument led out from a channel opening, near the lesion part in a substantially horizontal state with respect to a mucosa of the lesion part.

Note that, in the ESD, a surgeon needs to move the knife in parallel to a muscle layer of a submucosa and perform dissection in order to prevent the knife from touching the muscle layer. In that case, the surgeon makes full use of hand operation to always dispose the distal end side of the insertion section in parallel to the mucosa of the lesion part.

On the other hand, in an endoscope for large intestine observation, if a bent shape of a bending section is small in diameter, there is fear that it is difficult to straighten a large intestine in a manipulation for pulling on and straightening the large intestine.

This is because, when the bending section is bent to hook the distal end portion side of the insertion section on a tissue in a body cavity, the distal end portion side is weakly hooked because the bending section has the bent shape small in diameter.

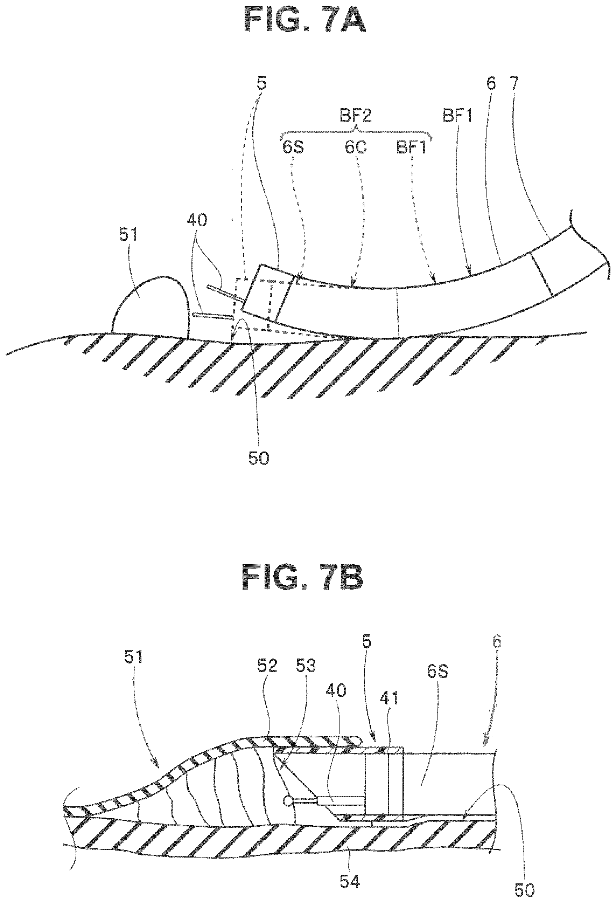

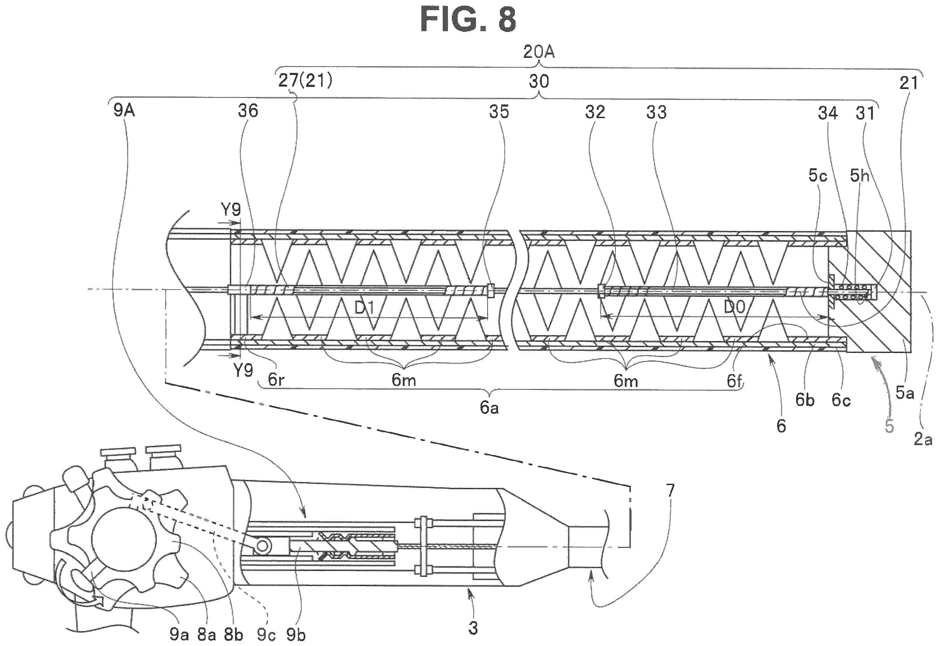

Japanese Patent No. 4856289 describes an endoscope including a configuration in which bending length can be changed in one bending section with a simple configuration. In Fig. 7 of Japanese Patent No. 4856289, a bent state in which the bending section is bent from a proximal end side of a second part is shown. In Fig. 8, a bent state in which the bending section is bent from a proximal end side of a first part is shown.

With the endoscope of Japanese Patent No. 4856289, by selectively switching the bent state shown in Fig. 7 and the bent state shown in Fig. 8, it is possible to adapt the endoscope to reverse operation in a large intestine, a manipulation for pulling on and straightening the large intestine, and the like.

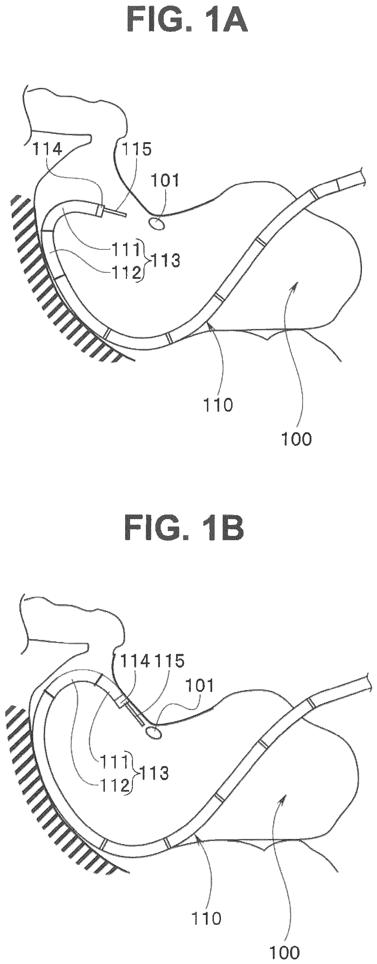

With the endoscope of Japanese Patent No. 4856289, when an insertion section 110 is inserted into a stomach 100 and a bending section 113 is bent from a proximal end side of a first part 111 as shown in Fig. 1A, a distal end portion 114 of the insertion section 110 separates from a lesion 101.

In this case, a sheath projection amount of a treatment instrument 115 increases to make it impossible to dispose the treatment instrument near a lesion part. This is because, even in a bent state in which a curvature of the bending section of the endoscope is large, flexibility is imparted to a sheath of the treatment instrument 115 such that the sheath can be smoothly inserted through a channel.

On the other hand, when the bending section 113 is bent from a proximal end side of a second part 112 as shown in Fig. 1B, it is possible to dispose the distal end portion 114 side of the insertion section 110 near the lesion. As a result, it is possible to project the sheath of the treatment instrument 115 from a distal end face of the distal end portion 114 by an appropriate amount. Thereafter, the ESD can be performed by performing hand operation for moving the distal end portion 114 in parallel along a mucosa of the lesion part.

That is, with the endoscope in which the bending length of the bending section 113 can be changed, it is possible to change the bent state of the bending section and dispose the distal end portion 114 of the insertion section 110 closer to the lesion part.

Note that, in the endoscope for large intestine observation, Japanese Patent Application Laid-Open Publication No. H09-084753 and the like describe a flexible adjustment mechanism that can increase rigidity of a flexible portion of the insertion section in order to insert the insertion section into a deeper part while maintaining the straightening.

However, in the endoscope of Japanese Patent No. 4856289, the bending section is bent from the first part or the second part. Therefore, it is difficult to dispose the distal end portion provided on the distal end portion side of the insertion section and a distal end side of the bending section in positions optimum for treatment along the mucosa near the lesion. Therefore, in the ESD, it is difficult to move the distal end portion located on the distal end side of the bent bending section along the mucosa near the lesion part.

Note that, in the ESD, when the sheath projection amount from the endoscope exceeds an appropriate value, a deficiency occurs in which the flexibility of the sheath yields to resistance of a lesion fiber layer and, in dissection, the sheath warps and sharpness is deteriorated.

When the resistance from the lesion fiber layer is large, even if a projection amount of the knife is optimum, there is fear that, when dissection by the knife is started, the bent state of the bending section changes to make it difficult to perform the ESD. This is because, in the dissection by the knife, reaction is applied to the knife from the lesion fiber layer, the reaction is transmitted to the distal end portion and the bending section on the distal end portion side via the sheath of the knife, and the bending section is bent in a direction opposite to a moving direction.

When a doctor with a lot of operation experience judges that the resistance from the lesion fiber layer is large, assuming that the reaction acts on the knife, the doctor performs twisting operation or the like of the insertion section while operating the bending knob and moves the distal end portion along the mucosa near the lesion part while maintaining the bent state to perform the ESD.

In other words, for an inexperienced doctor, it takes a lot of time to learn a technique for disposing the distal end portion of the insertion section near the lesion as determined in advance and learn a technique for, when the resistance from the lesion fiber layer is large, performing the ESD while maintaining the bent state of the insertion section with the hand operation.

The present invention has been devised in view of the above circumstances and an object of the present invention is to provide an endoscope in which rigidity on a distal end side of a bending section can be selectively changed according to an observation or a manipulation.

### Disclosure of Invention

### Means for Solving the Problem

An endoscope according to an aspect of the present invention includes: a bending section provided in an insertion section inserted into a subject, the bending section being bendable; and a switching section capable of changing rigidity on a distal end side of the bending section.

### Brief Description of the Drawings

Fig. 1A is a diagram for explaining a disposition example of a distal end portion in a state in which a bending section is bent from a proximal end side of a first part in an endoscope in which bending length can be changed;
Fig. 1B is a diagram for explaining a disposition example of the distal end portion in a state in which the bending section is bent from a proximal end side of a second part in the endoscope in which the bending length can be changed;
Fig. 2 is a diagram for explaining an endoscope including a bending section according to a first embodiment in which a pipe-like member of the present invention is provided;
Fig. 3 is a diagram for explaining a switching section according to the first embodiment;
Fig. 4A is a diagram for explaining an uncompressed state of a coupling tube;
Fig. 4B is an arrow Y4B-Y4B line sectional view of Fig. 4A;
Fig. 4C is a diagram for explaining a compressed state of the coupling tube;
Fig. 4D is an arrow Y4D-Y4D line sectional view of Fig. 4C;
Fig. 4E is a diagram for explaining a manufacturing example of the coupling tube by laser machining;
Fig. 5 is a diagram for explaining a rigidity switching operation device according to the first embodiment;
Fig. 6A is a diagram for explaining a relation between the rigidity switching operation device and the coupling tube according to the first embodiment;
Fig. 6B is a diagram for explaining a bent shape of the bending section in the uncompressed state of the coupling tube according to the first embodiment;
Fig. 6C is a diagram for explaining a bent shape of the bending section in the compressed state of the coupling tube in the first embodiment;
Fig. 7A is a diagram for comparing a state in which the bending section is bent in the uncompressed state of the coupling tube and a state in which the bending section is bent in the compressed state of the coupling tube according to the first embodiment;
Fig. 7B is a diagram for explaining endoscopic submucosal dissection performed by setting the bending section according to the first embodiment in a second bent shape including a substantially straight section;
Fig. 8 is a diagram for explaining a configuration example in which a coupling tube is provided on a flexible tube section side of the bending section in addition to a distal end portion side of the bending section;
Fig. 9 is an arrow Y9-Y9 line sectional view of Fig. 8 and is a diagram for explaining an attachment example of a fourth stopper;
Fig. l0A is a diagram for explaining a relation between the rigidity switching operation device and the coupling tube in a configuration example shown in Fig. 8;
Fig. 10B is a diagram for explaining a relation between the rigidity switching operation device and the coupling tube in a first restricted state in the configuration example shown in Fig. 8;
Fig. 10C is a diagram for explaining a relation between the rigidity switching operation device and the coupling tube in a second restricted state in the configuration example shown in Fig. 8;
Fig. 11A is a diagram for explaining a bent shape of the bending section in the compressed state of a first coupling tube and the uncompressed state of a second coupling tube in the configuration example shown in Fig. 8;
Fig. 11B is a diagram for explaining a bent shape of the bending section in an intermediate compressed state different from the compressed state of the first coupling tube and the second coupling tube in the configuration example shown in Fig. 8;
Fig. 11C is a diagram for explaining a bent shape of the bending section in the uncompressed state of the first coupling tube and the compressed state of the second coupling tube in the configuration example shown in Fig. 8;
Fig. 12 is a diagram for explaining an element wire configuring a spiral tube and having a characteristic in a sectional shape;
Fig. 13 is a diagram for explaining an endoscope in which the spiral tube formed of the element wire shown in Fig. 12 is provided in a bending section center;
Fig. 14 is a diagram for mainly explaining a bending section of an insertion section according to a second embodiment;
Fig. 15 is a diagram for explaining an operation section according to the second embodiment;
Fig. 16A is a diagram for explaining a bent shape of the bending section according to the second embodiment;
Fig. 16B is a diagram for explaining a bent shape of the bending section according to the second embodiment;
Fig. 17 is a diagram for explaining the bending section in a configuration example in which a coupling tube is provided on a flexible tube section side of the bending section in addition to a distal end side of the bending section;
Fig. 18A is a diagram for explaining an operation section according to a configuration example shown in Fig. 17;
Fig. 18B is a diagram for explaining a portion indicated by an arrow 18B in Fig. 18;
Fig. 18C is an arrow Y18C-Y18C line sectional view of Fig. 18B;
Fig. 19A is a diagram for explaining a bent shape of a bending section according to the configuration example shown in Fig. 17;
Fig. 19B is a diagram for explaining a bent shape of the bending section according to the configuration example shown in Fig. 17;
Fig. 19C is a diagram for explaining a bent shape of the bending section according to the configuration example shown in Fig. 17; and
Fig. 20 is a diagram showing an endoscope including a spiral tube in a proximal end side bending portion of the bending section.

### Best Mode for Carrying Out the Invention

Embodiments of the present invention are explained below with reference to the drawings.

Note that, in the respective figures referred to in the following explanation, in order to show respective components in sizes recognizable on the drawings, scales are sometimes differentiated for each of the components. That is, the present invention is not limited only to the numbers of the components described in the figures, shapes of the components, ratios of sizes of the components, and relative positional relations of the respective components.

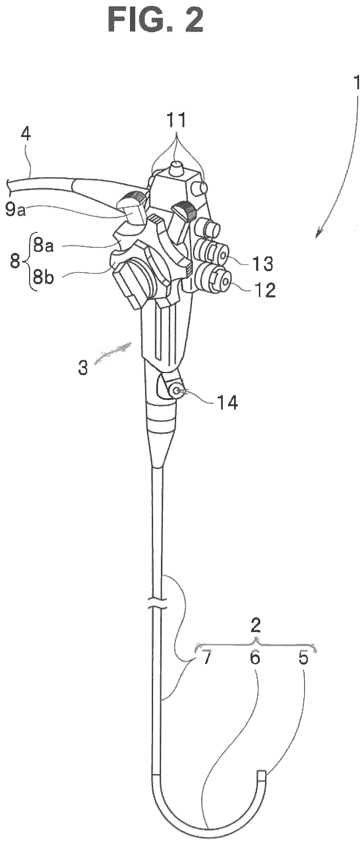

A first embodiment is explained with reference to Fig. 2 to Fig. 7B.

As shown in Fig. 2, an endoscope 1 mainly includes an insertion section 2, an operation section 3 provided on a proximal end side of the insertion section 2, and a universal cord 4 extended from a side portion of the operation section 3.

An endoscope connector (not shown in the figure) is provided at a proximal end portion of the universal cord 4. The endoscope connector is electrically connected to an external apparatus including a light source and a camera control unit.

The insertion section 2 of the endoscope 1 is inserted into a subject and adapted to an observation and a manipulation. The insertion section 2 is configured by concatenating, in order from a distal end side, a rigid distal end portion 5, a bending section 6 bendable in, for example, upward, downward, left, and right directions that makes it possible to direct the distal end portion 5 in a desired direction, and a flexible tube section 7 having flexibility.

On a side surface of the operation section 3 of the endoscope 1, an up-down knob 8a and a left-right knob 8b are provided as a bending section operation device 8 for bending the bending section 6. Reference sign 9a denotes a rigidity switching lever explained below (hereinafter abbreviated as switching lever). The switching lever 9a changes rigidity of a pipe-like member explained below provided in the bending section 6.

Various operation switches 11, an air/water feeding button 12 and a suction button 13, which are fluid control valves for endoscope, and the like are provided in the operation section 3.

Note that reference numeral 14 denotes a treatment instrument insertion port. A treatment instrument for endoscope such as grasping forceps is inserted into the treatment instrument insertion port 14. On a distal end face of the distal end portion 5, an illumination window (not shown in the figure) of an illumination optical system that illuminates a region to be examined, an observation window (not shown in the figure) of an image pickup optical system that picks up an image of the illuminated region to be examined, a nozzle (not shown in the figure) that ejects fluid for removing body fluid and the like adhering to the observation window or the illumination window, a channel opening (not shown in the figure), which is a distal end opening of the treatment instrument channel (not shown in the figure), and the like are provided.

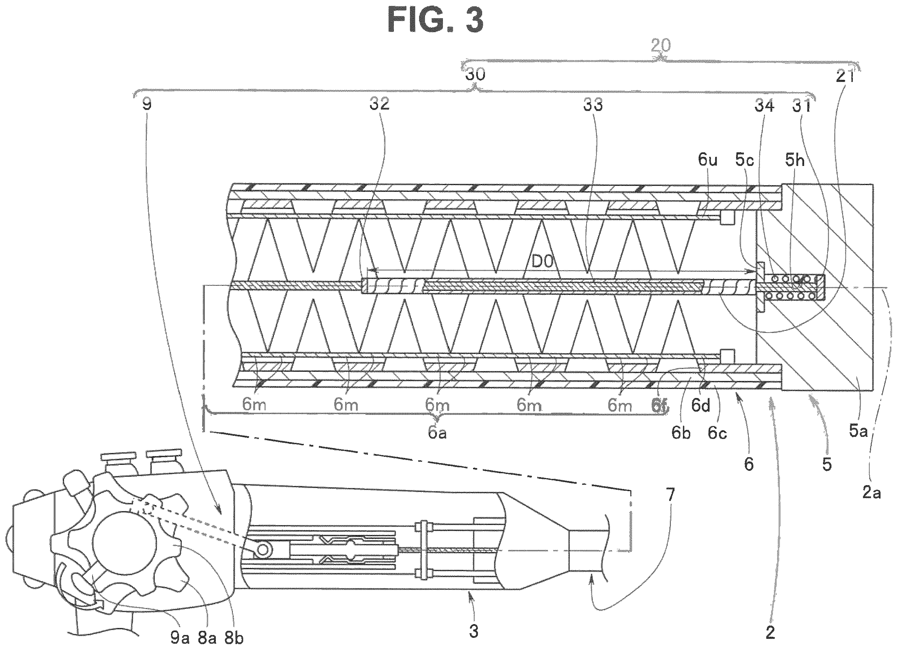

Configurations of the distal end portion 5 and the bending section 6 are explained with reference to Fig. 3.

As shown in Fig. 3, an upward bending wire 6u, a downward bending wire 6d, a left bending wire (not shown in the figure), and a right bending wire (not shown in the figure) corresponding to bending directions are provided in the bending section 6. Reference numeral 5a denotes a distal end rigid portion. The distal end rigid portion 5a configures the distal end portion 5.

In the distal end rigid portion 5a, a setting hole 5h is provided as a concave section including an opening, for example, in a center on a proximal end face side in addition to an axial direction through-hole for illumination (not shown in the figure) in which an illumination unit (not shown in the figure) is disposed, an axial direction through-hole for image pickup (not shown in the figure) in which an image pickup unit (not shown in the figure) is disposed, an axial direction through-hole for channel (not shown in the figure) including a distal end opening (not shown in the figure) from which a treatment instrument is led out, and an axial direction through-hole for air/water feeding in which a cleaning nozzle (not shown in the figure) is provided.

A position of a bottom surface of the setting hole 5h is set to be a predetermined position on a distal end side of the insertion section 2.

Note that it is also possible to provide an opening of the setting hole on a distal end face side and set a proximal end side end face of a lid member, which closes the opening and configures the bottom surface of the setting hole, as the predetermined position on the distal end side of the insertion section 2.

The bending section 6 includes a predetermined bending piece set 6a in which a distal end bending piece 6f, a plurality of intermediate bending pieces 6m, and a proximal end bending piece (not shown in the figure) are turnably concatenated.

An outer circumference of the bending piece set 6a is covered by a braid 6b, which is a mesh-like tube made of metal. An outer circumference of the braid 6b is further covered by a bending tube 6c. As a result, the bending section 6 bending in a predetermined bent shape is configured.

Note that the bending tube 6c is a tube made of rubber such as fluororubber.

Respective distal ends of the upward, downward, left, and right bending wires are fixed in predetermined positions corresponding to top, bottom, left, and right of an inner surface of the distal end bending piece 6f. The respective bending wires are extended into the operation section 3 passing through the insertion section 2.

Reference numeral 20 denotes a switching section. The switching section 20 is configured by a coupling tube 21 and a coupling tube operation section 30.

The coupling tube 21 is disposed in a predetermined position on an inside of the bending section 6.

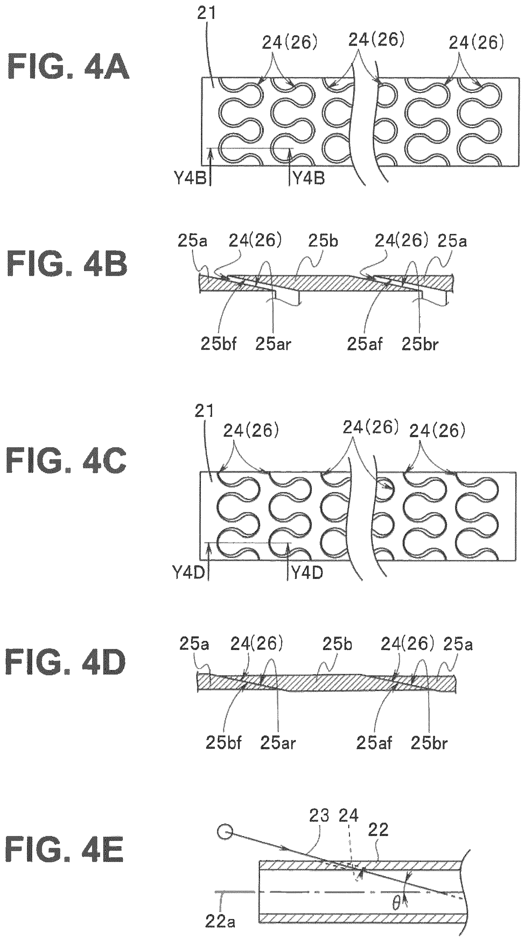

The coupling tube 21 shown in Fig. 4A and Fig. 4C is a pipe-like member that can be switched to a compressed state and an uncompressed state with respect to an insertion axial direction of the insertion section 2. The bending section 6 bends into a predetermined bent shape in the uncompressed state of the coupling tube 21.

On the other hand, the bending section 6 less easily bends in the compressed state of the coupling tube 21 and the bending section 6 is substantially straightened.

That is, in the uncompressed state, the coupling tube 21 has first rigidity at which the coupling tube 21 easily bends. In the compressed state, the coupling tube 21 has second rigidity at which the coupling tube 21 less easily bends.

The coupling tube 21 in the present embodiment is a laser-cut pipe. The coupling tube 21 is configured by forming separation and connection grooves 24 having a predetermined shape one by one by radiating a laser beam 23 on the rigid tube 22 as shown in Fig. 4E.

The laser-cut pipe is a tubular member connected without being separated to come off by the separation and connection grooves 24 as shown in Fig. 4A to Fig. 4D.

As shown in Fig. 4E, the coupling tube 21 alternately includes a first joint piece 25a, which is a first annular section, separated by obliquely radiating the laser beam 23 on an outer circumferential surface of the rigid tube 22 and a second joint piece 25b, which is a second annular section. More specifically, the laser beam 23 tilts at a predetermined angle θ (θ is an acute angle) with respect to a center axis 22a of the rigid tube 22.

As shown in Fig. 4B and Fig. 4D, the joint pieces 25a, 25b, 25a, ... adjacent to one another of the coupling tube 21 are configured to be capable of sliding in the insertion axial direction and not to come off by placing a distal end side inclined surface 25bf, which inclines with respect to the center axis 22a of the second joint piece 25b, on a proximal end side inclined surface 25ar, which inclines with respect to the center axis 22a of the first joint piece 25a, and placing a distal end side inclined surface 25af, which inclines with respect to the center axis 22a of the first joint piece 25a, on a proximal end side inclined surface 25br, which inclines with respect to the center axis 22a of the second joint piece 25b.

In the coupling tube 21, the joint pieces 25a and 25b overlap little when a separation distance by gaps 26 formed by the separation and connection grooves 24 is sufficiently provided between the adjacent joint pieces 25a and 25b as shown in Fig. 4A and Fig. 4B. In other words, an adhesion amount of the inclined surfaces 25ar and 25br and an adhesion amount of the inclined surfaces 25br and 25f of the joint pieces 25a and 25b decrease. Then, the coupling tube 21 is in an uncompressed state and is formed as a low-harness tube having predetermined flexibility.

On the other hand, when the gaps 26 formed by the separation and connection grooves 24 are narrowed as shown in Fig. 4C and Fig. 4D and the adhesion amount of the inclined surfaces 25ar and 25bf and the adhesion amount of the inclined surfaces 25br and 25f of the joint pieces 25a and 25b increases, the coupling tube 21 is in a compressed state.

The coupling tube 21 in the compressed state has a straight shape. Rigidity of the coupling tube 21 is high compared with the coupling tube 21 in the uncompressed state. The coupling tube 21 is less easily bent. At this point, the gaps 26 between the adjacent pieces of the coupling tube 21 are narrowed and the separation distance decreases, whereby entire length of the coupling tube 21 decreases compared with the uncompressed state. That is, the coupling tube 21 is compressed, whereby the gaps 26 are gradually narrowed compared with the uncompressed state and the entire length of the coupling tube 21 decreases.

As the gaps 26 between the adjacent joint pieces 25a and 25b are narrowed, an amount of overlapping (also referred to as adhesion amount) of the inclined surface 25af and 25bf increases when the coupling tube 21 is bent. The inclined surfaces 25af and 25bf come into contact with (adhere to) each other, whereby contact resistance is generated. The contact resistance increases according to the increase in the adhesion amount. The coupling tube 21 is less easily bent.

In this way, by setting the separation distance between the adjacent joint pieces 25a and 25b as appropriate, the coupling tube 21 is changed to desired rigidity, in other words, a predetermined easily bendable state or a predetermined less easily bendable state.

In the present embodiment, in the coupling tube 21 disposed in the bending section 6, the gaps are set such that the coupling tube 21 has flexibility for not causing hindrance to bending performance of the bending section 6 in the uncompressed state.

When the total length is D0, the coupling tube 21 in the present embodiment changes to a less easily bendable compressed state. When the total length is D1, the coupling tube 21 changes to the easily bendable uncompressed state.

Note that, in general, the rigid tube 22 is made of stainless steel. However, the rigid tube 22 may be made of a nickel titanium alloy.

As shown in Fig. 3, the switching section 20 is configured by the coupling tube 21 described above and the coupling tube operation section 30. The coupling tube operation section 30 includes a first stopper 31, a second stopper 32, an operation wire 33, a pressing member 34, and a rigidity switching operation device 9 provided in the operation section 3.

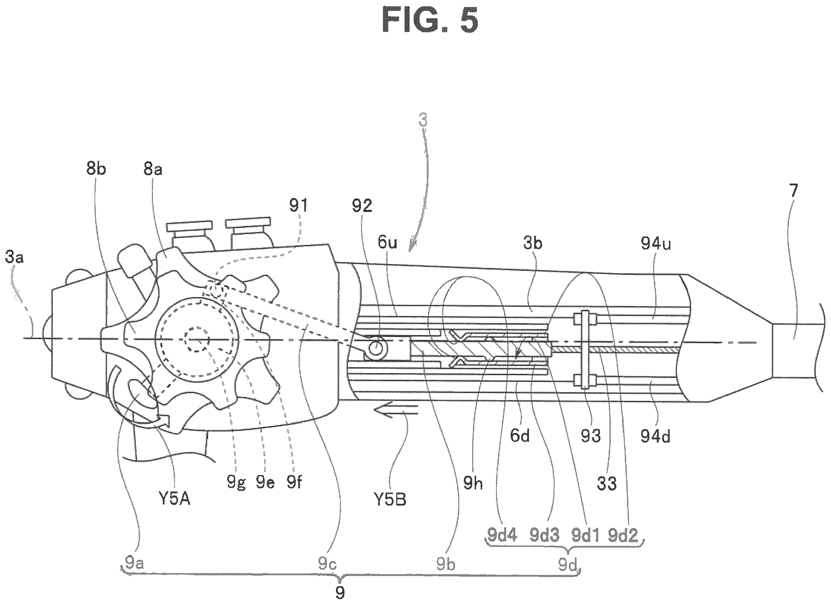

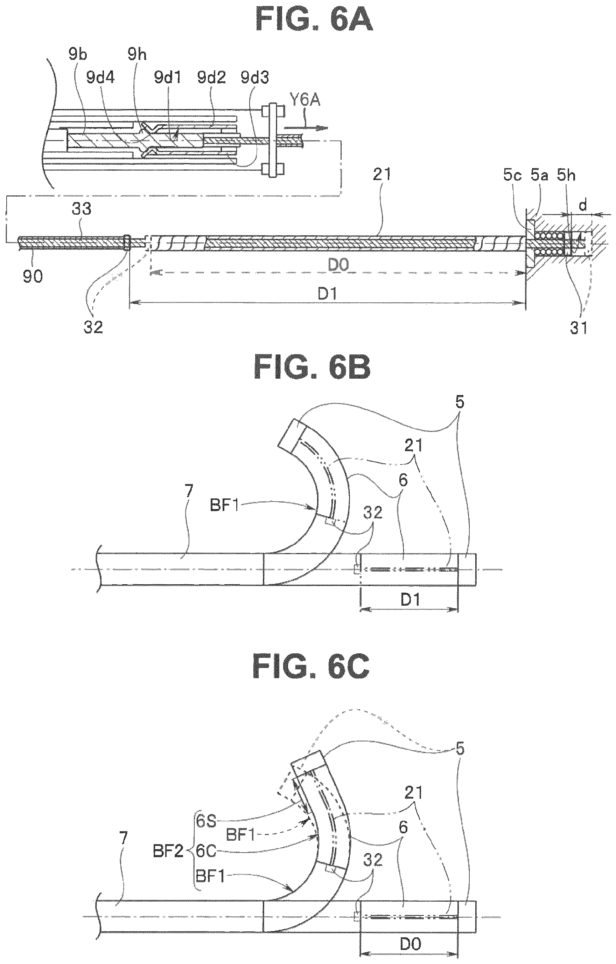

The operation wire 33 is inserted through a through-hole of the coupling tube 21. The operation wire 33 is a towing member and is set to predetermined length. In the present embodiment, the operation wire 33 is inserted through the insertion section 2 from the setting hole 5h of the distal end rigid portion 5a and extends to a slide member (see reference sign 9b in Fig. 5) explained below of the operation section 3. The operation wire 33 is inserted through a coil sheath for operation (see reference numeral 90 in Fig. 6A) and led out into the operation section 3.

The operation wire 33 is optimally disposed along a longitudinal center axis 2a of the insertion section 2 not to change a bending angle of the bending section 6 when the operation wire 33 is towed. In the present embodiment, the operation wire 33 is disposed substantially along the longitudinal center axis 2a in a substantial center portion in the bending section 6. As a result, the coupling tube 21 is disposed along the longitudinal center axis 2a.

The first stopper 31 and the second stopper 32, which are restricting sections, are made of, for example, metal and integrally fixed by adhesion or bonding in predetermined positions of the operation wire 33.

The first stopper 31 is fixedly disposed to a distal end portion of the operation wire 33. The second stopper 32 is fixedly disposed to a halfway portion of the operation wire 33.

A distal end face of the second stopper 32 sets a disposition position of an end face on a proximal end side of the coupling tube 21 inserted through the operation wire 33.

In the present embodiment, a distal end face of the coupling tube 21 and a proximal end face of a lid body 5c explained below are integrally fixed. A proximal end face of the coupling tube 21 and the distal end face of the second stopper 32 are integrally fixed.

The first stopper 31 is disposed to be capable of sliding in the axial direction in the setting hole 5h.

Note that, when the operation wire 33 is prevented from turning around an axis, a sectional shape of the first stopper 31 is formed in, for example, a square shape different from a circular shape. The setting hole 5h is also formed in the same shape.

In the setting hole 5h, a compression spring such as a coil spring, which is the pressing member 34, is disposed in the setting hole 5h. The operation wire 33 is inserted through a through-hole of the coil spring. Therefore, the pressing member 34, the lid body 5c, and the coupling tube 21 are disposed between the first stopper 31 and the second stopper 32.

The pressing member 34 has a predetermined urging force (also referred to as elastic force). A distal end face of the first stopper 31 disposed in the setting hole 5h is disposed to be pressed against a bottom surface of the setting hole 5h by an expanding elastic force of the pressing member 34.

A proximal end face side opening of the setting hole 5h is closed by the lid body 5c. A through-hole (not shown in the figure), through which the operation wire 33 is inserted, or a cutout groove (not shown in the figure), in which the operation wire 33 is disposed, is formed in the lid body 5c.

The lid body 5c closes the proximal end face side opening of the setting hole 5h to prevent the pressing member 34 from coming off the setting hole 5h. The distal end face of the coupling tube 21, through which the operation wire 33 is inserted as explained above, is integrally fixed to a proximal end face of the lid body 5c.

In a state shown in the figure in which the distal end face of the first stopper 31 is disposed to be pressed against the bottom surface of the setting hole 5h by the pressing member 34, a distance from the proximal end face of the lid body 5c to the distal end face of the second stopper 32 is D0 (see Fig. 3 and Fig. 6A).

At this point, length of the coupling tube 21 disposed between the lid body 5c and the second stopper 32 is also D0. The coupling tube 21 is in the predetermined compressed state.

The proximal end face of the coupling tube 21 is disposed in a position for dividing the bending section 6 into a distal end side and a proximal end side or further on the distal end portion 5 side than the position. In other words, the coupling tube 21 is provided in a distal end side bending portion of the bending section 6.

Note that the compressed state of the coupling tube 21 is set to flexibility (rigidity) capable of preventing the distal end portion 5 side of the bending section 6 from easily bending and forming a straight portion on the distal end portion 5 side of the bending section 6.

In the present embodiment, when a towing force for towing the wire 33 exceeds the urging force of the pressing member 34, the operation wire 33 is towed in an operation section direction. The operation wire 33 is towed, whereby the first stopper 31 and the second stopper 32 fixedly disposed to the operation wire 33 are integrally moved to the operation section 3 side.

Then, the pressing member 34 is gradually retracted according to the movement of the first stopper 31. The distance from the distal end face of the second stopper 32 to the proximal end face of the lid body 5c gradually increases to be larger than D0 according to the movement of the second stopper 32. Therefore, the coupling tube 21 is gradually shifted from the predetermined compressed state to the uncompressed state.

The distance from the proximal end face of the lid body 5c to the distal end face of the second stopper 32 increases to D1 (see Fig. 6A), thereby the coupling tube 21 changes to the uncompressed state in which the length is D1.

A configuration of the rigidity switching operation device 9 is explained with reference to Fig. 5.

As shown in Fig. 5, the rigidity switching operation device 9 mainly includes a switching lever 9a, a link mechanism, and a slide-member holding section 9k. The link mechanism includes the slide member 9b disposed to be capable of advancing and retracting in the operation section 3 and a driving-force transmitting member 9c.

The switching lever 9a is capable of turning. On the other hand, the slide member 9b advances or retracts along an operation section longitudinal axis 3a in a sliding groove 9d1 according to turning operation of the switching lever 9a. The driving-force transmitting member 9c is, for example, an elongated flat member. The driving-force transmitting member 9c transmits a rotating motion of the switching lever 9a to the slide member 9b and advances and retracts the slide member 9b in a longitudinal axis direction.

The slide-member holding section 9k is fixedly disposed to a base plate 3b provided in the operation section 3. The sliding groove 9d1, in which the slide member 9b is disposed to be capable of advancing and retracting, is provided in the slide-member holding section 9k.

The sliding groove 9d1 is configured by leaf springs 9d2 and 9d3 erected in a pair of opposed positions. Locking convex sections 9d4 functioning as locking sections are provided in predetermined positions of respective lever side end portions of the leaf springs 9d2 and 9d3 in an opposed positional relation.

The switching lever 9a is integral with a lever main body 9e, which is a disk. A coupling protrusion 9f is provided in a predetermined position of the lever main body 9e. The coupling protrusion 9f is a convex portion projecting to an outer side from an outer circumferential surface of the lever main body 9e.

Reference sign 9g is a shaft body. The shaft body 9g rotates clockwise or counterclockwise. The lever main body 9e is integrally fixedly disposed to the shaft body 9g. Therefore, the lever main body 9e is configured to rotate around an axis of the shaft body 9g according to rotation operation of the switching lever 9a.

One end portion of the driving-force transmitting member 9c is turnably axially supported in the coupling protrusion 9f by a first pin 91. The other end portion of the driving-force transmitting member 9c is turnably axially supported at one end portion of the slide member 9b by the second pin 92.

A proximal end portion of the operation wire 33 is integrally fixedly disposed to the other end portion of the slide member 9b. A slide member convex section 9h, which is an outer flange functioning as a locking section, is provided in a predetermined position in a halfway portion of the slide member 9b.

Reference numeral 93 denotes a coil-sheath fixing section. The coil-sheath fixing section 93 is fixed to the base plate 3b of the operation section 3. Proximal end portions of coil sheaths 94u and 94d are fixedly disposed to the coil-sheath fixing section 93. The upward bending wire 6u is extended from the coil sheath 94u for upward operation. The downward bending wire 6d is extended from the coil sheath 94d for downward operation.

A proximal end of the upward bending wire 6u is fixed to one end portion of a chain (not shown in the figure) that meshes with a sprocket (not shown in the figure) integrally coupled and fixed to a shaft (not shown in the figure) of the up-down knob 8a. A proximal end of the downward bending wire 6d is fixed to the other end portion of the chain that meshes with the sprocket as explained above.

Action of the rigidity switching operation device 9 is explained with reference to Fig. 3, Fig. 5, and Fig. 6A to Fig. 6C.

When a user operates the switching lever 9a in an arrow Y5A direction in Fig. 5, the slide member 9b is moved in an arrow Y5B direction. Towing of the operation wire 33 is started.

Thereafter, the moved slide member convex section 9h comes into contact with the locking convex sections 9d4 of the leaf springs 9d2 and 9d3. The slide member 9b is further moved in an arrow Y5B direction, whereby the leaf springs 9d2 and 9d3 are elastically deformed to expand to an operation section side portion outer side by the moving slide member convex section 9h.

The slide member convex section 9h moves while being in contact with the locking convex sections 9d4, whereby an interval between the locking convex sections 9d4 is further increased. The slide member convex section 9h passes between the locking convex sections 9d4. Then, after the passage, the elastically deformed leaf springs 9d2 and 9d3 return to original states.

As a result, as shown in Fig. 6A, the slide member convex section 9h is held by the locking convex sections 9d4 of the leaf springs 9d2 and 9d3 and restricted from moving in an arrow Y6A direction.

At this point, the first stopper 31 and the second stopper 32 integrally fixed to the towed operation wire 33 move as explained above. That is, the distal end face of the first stopper 31 separates from the bottom surface of the setting hole 5h by a distance d. On the other hand, a disposition position of the distal end face of the second stopper 32 is a position where the distance from the proximal end face of the lid body 5c is D1. As a result, the coupling tube 21 is switched from the compressed state to the uncompressed state.

In the uncompressed state, a position of the slide member convex section 9h is restricted by the locking convex sections 9d4. Therefore, the coupling tube 21 is retained in the uncompressed state even if the user detaches the switching lever 9a.

In the uncompressed state of the coupling tube 21, when the user rotates the up-down knob 8a, the bending wire 6u is towed and the bending section 6 bends in a predetermined basic bent shape (hereinafter referred to as first bent shape) BF1 as shown in Fig. 6B.

Switching from the uncompressed state to the compressed state of the coupling tube 21 is explained.

When switching the coupling tube 21 from the uncompressed state to the compressed state, the user operates the switching lever 9a in an opposite direction of the arrow Y5A shown in Fig. 5. Then, the slide member 9b is moved in an opposite direction of the arrow Y5B according to rotation of the switching lever 9a. The restriction of the slide member convex section 9h by the locking convex sections 9d4 is released.

Thereafter, the first stopper 31 and the second stopper 32 are moved by rotation operation of the switching lever 9a and an urging force of the pressing member 34.

As a result, the distal end face of the first stopper 31 is disposed in contact with the setting hole 5h again. On the other hand, the disposition position of the distal end face of the second stopper 32 returns to the position where the distance from the proximal end face of the lid body 5c is D0. As a result, the coupling tube 21 is switched from the uncompressed state to the compressed state.

In the compressed state, when the user rotates the up-down knob 8a, the bending wire 6u is towed. As indicated by a broken line in Fig. 6C, the bending section 6 bends in a deformed bent shape (hereinafter, second bent shape) BF2 different from the first bent shape BF1 shown in Fig. 6B.

In a second bent shape BF2, the coupling tube 21 is less easily bent in the compressed state compared with the uncompressed state. The second bent shape BF2 is formed to exhibit a function of a stylet.

The function of the stylet is a function of, in endotracheal intubation or the like, inserting a bar-like stylet more rigid than a soft intubation tube bent in a bow shape into an endotracheal tube of the intubation tube to prevent the intubation tube from being bent by insertion resistance to make it easy to intubate.

That is, in a proximal end side bending portion on the flexible tube section 7 side of the bending section 6, the second bent shape BF2 generally bends in the first bent shape BF1 substantially the same as the uncompressed state.

On the other hand, on the distal end portion 5 side of the bending section 6, that is, in the distal end side bending portion, the second bent shape BF2 changes to a bent shape different from the first bent shape BF1 from a vicinity of a position where the second stopper 32 is disposed toward the distal end portion 5 side.

More specifically, on a distal end side of the bending section 6, a substantially straight section 6S appears continuously to the distal end portion 5. Therefore, a curved section 6C having a curvature smaller than a curvature of the first bent shape BF1 appears in a range from a proximal end of the substantially straight section 6S to a vicinity of the second stopper 32.

That is, the second bent shape BF2 is a bent shape including the substantially straight section 6S, the curved section 6C, and the first bent shape BF1 in order from the distal end portion 5 side.

In the configuration explained above, when the coupling tube 21 is bent in the uncompressed state, the bending section 6 bends in the first bent shape BF1 as indicated by a solid line in Fig. 7A. Therefore, it is difficult to lead out a treatment instrument 40, which is led out from a channel opening (not shown in the figure), substantially in parallel to a mucosa surface 50 and operate the treatment instrument 40 toward a lesion 51.

On the other hand, when bending operation is performed after the coupling tube 21 is switched to the compressed state, the bending section 6 is bent in the second bent shape BF2 including the substantially straight section 6S as indicated by a broken line in Fig. 7A. Therefore, it is possible to dispose the substantially straight section 6S near the lesion 51 along the mucosa surface 50. As a result, it is possible to perform endoscopic mucosal dissection shown in Fig. 7B.

More specifically, the distal end portion 5 can be disposed near the lesion 51 with the substantially straight section 6S of the bending section 6 set along the mucosa surface 50. Therefore, after the lesion 51 is swelled, it is possible to perform dissection in a state in which a hood 41 attached to the distal end portion 5 is inserted under a mucosa 52 of the lesion 51 to pull a fiber layer 53 as shown in Fig. 7B.

When an IT knife is used as the treatment instrument 40, it is possible to move the treatment instrument 40 in parallel and set a lead-out state at an optimum distance without hurting a muscle layer 54 when the treatment instrument 40 is led out from the hood 41 toward the fiber layer 53.

Thereafter, hand operation for moving the distal end portion 5 in parallel to the muscle layer 54 is performed to dissect the fiber layer 53 in a state in which the fiber layer 53 is pulled by the treatment instrument 40. At this point, since the coupling tube 21 has rigidity that prevents the coupling tube 21 from easily bending in the compressed state, it is possible to perform the dissection without a bent shape of the bending section 6 changing during the dissection by the knife.

In this way, in the endoscope 1, the switching section 20 configured by the coupling tube 21, which can be switched to the uncompressed state and the compressed state, provided on the distal end portion 5 side of the bending section 6 and the coupling tube operation section 30, which switches the coupling tube 21 to the compressed state or the uncompressed state, is provided. As a result, it is possible to selectively perform hand operation of the switching lever 9a to switch rigidity on the distal end portion 5 side of the bending section 6 to perform observation or treatment.

By forming the bending section 6 in the second bent shape BF2 including the substantially straight section 6S, it is possible to smoothly perform treatment such as dissection by the IT knife.

Note that, in the embodiment, the coupling tube 21 that can be switched to the uncompressed state and the compressed state is provided on the distal end portion 5 side of the bending section 6.

However, a second coupling tube 21 that can be switched to the uncompressed state and the compressed state may be provided on the flexible tube section 7 side of the bending section 6 in addition to the distal end portion 5 side of the bending section 6 as shown in Fig. 8.

A configuration example in which coupling tubes are provided in the distal end side bending portion and the proximal end side bending portion of the bending section is explained with reference to Fig. 8 to Fig. 11C.

In the following explanation, the coupling tube 21 provided on the distal end portion 5 side of the bending section 6 is described as first coupling tube 21 and the coupling tube 21 provided on the flexible tube section 7 side of the bending section 6 is described as second coupling tube 27. Members same as the members in the embodiment explained above are denoted by the same reference numerals and explanation of the members is omitted.

As shown in Fig. 8, a switching section 20A in the present embodiment includes a third stopper 35 and a fourth stopper 36 functioning as restricting sections, the second coupling tube 27, and a rigidity switching operation device 9A provided in the operation section 3 in addition to the first coupling tube 21, the first stopper 31, the second stopper 32, the operation wire 33, and the pressing member 34.

Note that reference sign 6r denotes a proximal end bending piece.

The third stopper 35 is made of metal like the second stopper 32. The third stopper 35 is integrally fixed to a predetermined position of the operation wire 33 by adhesion or bonding. The third stopper 32 is disposed in a halfway portion of the operation wire 33 and further on the flexible tube section 7 side than a position for dividing the bending section 6 into a distal end side and a proximal end side.

A proximal end face of the third stopper 35 sets a disposition position of a distal end face of the second coupling tube 27 inserted through the operation wire 33. In the present embodiment, the proximal end face of the third stopper 35 and the distal end face of the second coupling tube 27 are integrally fixed.

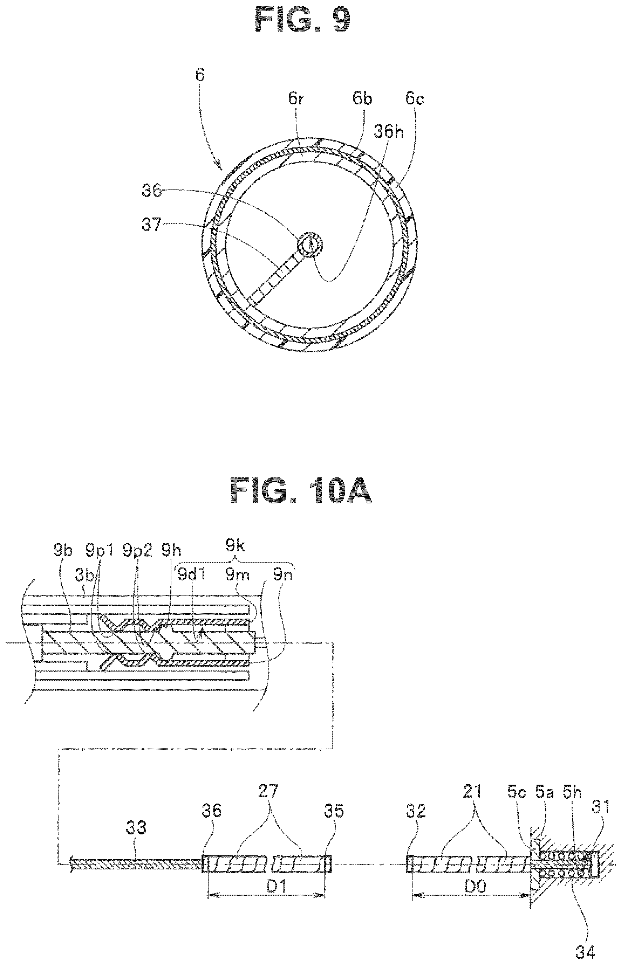

The fourth stopper 36 is made of metal. As shown in Fig. 9, the fourth stopper 36 is fixedly disposed to a center of the proximal end bending piece 6r via a support plate 37. One end portion of the support plate 37 is bonded to an inner circumferential surface of the proximal end bending piece 6r. The fourth stopper 36 is bonded to the other end portion of the support plate 37. The operation wire 33 is disposed in a through-hole 36h of the fourth stopper 36.

A distal end face of the fourth stopper 36 sets a disposition position of a proximal end face of the second coupling tube 27. In the present embodiment, the distal end face of the fourth stopper 36 and the proximal end face of the second coupling tube 27 are integrally fixed.

As shown in Fig. 8, whereas a distance from the proximal end face of the lid body 5c to the distal end face of the second stopper 32 is D0, a distance from the distal end face of the fourth stopper 36 to the proximal end face of the third stopper 35 is set to D1.

Therefore, whereas the first coupling tube 21 in the bending section 6 is in the compressed state explained above, the second coupling tube 27 is in the uncompressed state explained above.

The rigidity switching operation device 9A is explained.

The rigidity switching operation device 9A in the present embodiment mainly includes, as shown in Fig. 8 and Fig. 10A, the switching lever 9a explained above, a link mechanism including the slide member 9b and the driving-force transmitting member 9c, and a slide-member holding section 9k including the sliding groove 9d1 in which the slide member 9b is disposed to be capable of advancing and retracting.

The slide-member holding section 9k is fixedly disposed to the base plate 3b provided in the operation section 3 like the slide-member holding section 9d explained above. In the slide-member holding section 9k, the sliding groove 9d1 in which the slide member 9b is disposed to be capable of advancing and retracting is provided.

In the present embodiment, the sliding groove 9d1 is configured by leaf springs 9m and 9n erected in a pair of opposed positions.

First locking convex sections 9p1 functioning as locking sections are provided in an opposed positional relation in predetermined positions of respective lever side end portions of the leaf springs 9m and 9n. In addition, second locking convex sections 9p2 functioning as locking sections are provided in an opposed positional relation in predetermined positions further on a leaf spring center side than respective lever side end portions of the leaf springs 9m and 9n.

That is, two locking convex sections 9p1 and 9p2 are provided in each of the leaf springs 9m and 9n. The first locking convex section 9p1 and the second locking convex section 9p2 are disposed in this order from the lever side.

Action of the rigidity switching operation device 9A is explained with reference to Fig. 8 and Fig. 10A to Fig. 11C.

The user operates the switching lever 9a and tows the operation wire 33, whereby the slide member convex section 9h comes into contact with the second locking convex sections 9p2 of the leaf springs 9m and 9n. The slide member 9b is further moved in the same direction, whereby the slide member convex section 9h passes between the second locking convex sections 9p2. After the passage, the elastically deformed leaf springs 9m and 9n return to the original state.

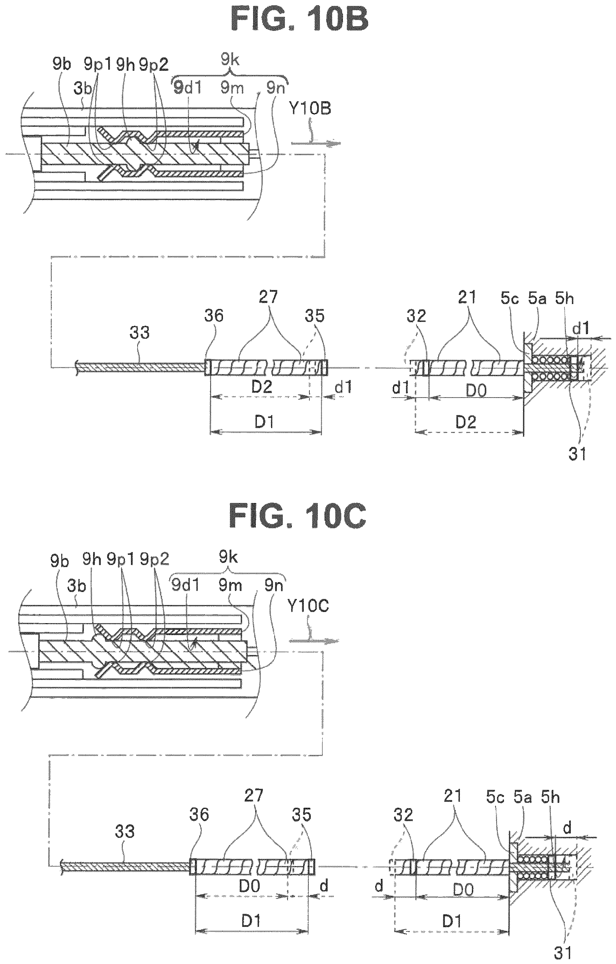

As a result, as shown in Fig. 10B, the slide member convex section 9h changes to a first restricted state in which the slide member convex section 9h is held by the second locking convex sections 9p2 of the leaf springs 9m and 9n and restricted from moving in an arrow Y10B direction.

In the first restricted state, the distal end face of the first stopper 31 fixedly disposed to the operation wire 33 separates from the bottom surface of the setting hole 5h by a distance d1.

As a result, in the disposition position of the distal end face of the second stopper 32, the distance from the proximal end face of the lid body 5c changes from D0 to D2. On the other hand, in the disposition position of the proximal end face of the third stopper 35, the distance from the distal end face of the fourth stopper 36 changes from D1 to D2.

That is, in the first restricted state, length of the first coupling tube 21 and the second coupling tube 27 changes to D2. The first coupling tube 21 and the second coupling tube 27 are switched to an intermediate compressed state different from the compressed state and the uncompressed state explained above.

Thereafter, the user further moves the slide member 9b in the same direction, whereby the slide member convex section 9h comes into contact with the first locking convex sections 9p1 of the leaf springs 9m and 9n. The slide member 9b is further moved in the same direction, whereby the slide member convex section 9h passes between the first locking convex sections 9p1. After the passage, the elastically deformed leaf springs 9m and 9n return to the original state.

As a result, as shown in Fig. 10C, the slide member convex section 9h changes to a second restricted state in which the slide member convex section 9h is held by the first locking convex sections 9p1 of the leaf springs 9m and 9n and movement in an arrow Y10C direction is restricted.

In the second restricted state, the distal end face of the first stopper 31 fixedly disposed to the operation wire 33 separates from the bottom surface of the setting hole 5h by the distance d.

As a result, the disposition position of the distal end face of the second stopper 32 changes to a position where the distance from the proximal end face of the lid body 5c is D1. On the other hand, the disposition position of the proximal end face of the third stopper 35 changes to a position where the distance from the distal end face of the fourth stopper 36 is D0.

That is, in the second restricted state, the first coupling tube 21 changes to the uncompressed state and the second coupling tube 27 changes to the compressed state.

Bending operation of the bending section 6 is explained.

First, as shown in Fig. 10A, when the first coupling tube 21 is in the compressed state and the second coupling tube 27 is in the uncompressed state, the bending section 6 less easily bends on the distal end portion 5 side.

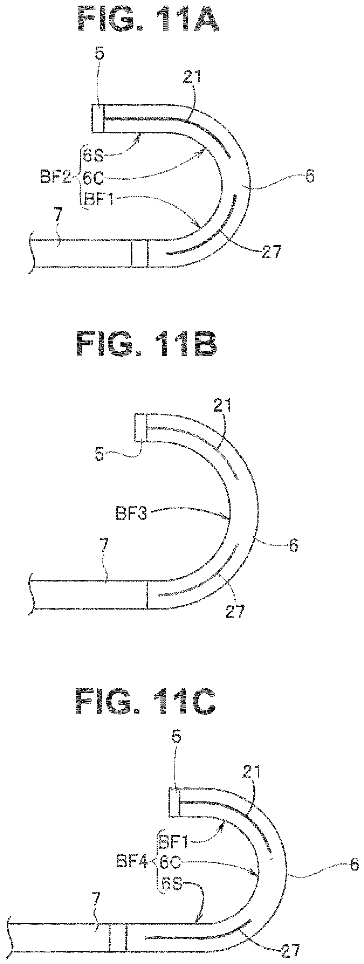

When the user rotates the up-down knob 8a, the bending wire 6u is towed. As shown in Fig. 11A, the bending section 6 bends in the second bent shape BF2 same as the second bent shape BF2 in the embodiment including, in order from the distal end portion 5 side, the substantially straight section 6S, the curved section 6C, and the first bent shape BF1.

Subsequently, in the first restricted state shown in Fig. 10B, as explained above, the first coupling tube 21 and the second coupling tube 27 are in the same intermediate compressed state.

Therefore, in the bending section 6, rigidity of the distal end side bending portion on the distal end portion 5 side and rigidity of the proximal end side bending portion on the flexible tube section 7 side are intermediate rigidity of the rigidity explained above. When the user rotates the up-down knob 8a, the bending wire 6u is towed. As shown in Fig. 11B, in the bending section 6, the distal end portion 5 side and the flexible tube section 7 side have substantially the same curvature. That is, the bending section 6 changes to a bent shape approximate to the first bent shape BF1, that is, a third bent shape BF3 in which a radius is set larger than a radius of the first bent shape BF1 and the entire bending section is equally bent.

Subsequently, in the second restricted state shown in Fig. 10C, the first coupling tube 21 changes to the uncompressed state and the second coupling tube 27 changes to the uncompressed state. Therefore, in the bending section 6, rigidity of the proximal end side bending portion is high.

Therefore, when the user rotates the up-down knob 8a, the bending wire 6u is towed. As shown in Fig. 11C, the bending section 6 bends in a fourth bent shape BF4 including the first bent shape BF1 on the distal end portion 5 side, including the substantially straight section 6S on the flexible tube section 7 side, and including the curved section 6C provided between the substantially straight section 6S and the first bent shape BF1.

In this way, in the endoscope 1, the switching section 20A configured by the coupling tubes 21 and 27, which can be switched to the uncompressed state and the compressed state, respectively provided in the distal end side bending portion and the proximal end side bending portion of the bending section 6 and the coupling tube operation section 30, which switches the coupling tubes 21 and 27 to the compressed state and the uncompressed state, is provided.

As a result, it is possible to selectively perform hand operation of the switching lever 9a to switch rigidity of the distal end side bending portion of the bending section 6 to high rigidity, intermediate rigidity, and low rigidity and change rigidity of the proximal end side bending portion of the bending section 6 to low rigidity, intermediate harness, and high rigidity.

In the endoscope explained above, when dissection by the IT knife is performed, the switching lever 9a is disposed in the first restricted state. Consequently, it is possible to obtain action and effects same as the action and the effects explained above.

Note that, in the endoscope explained above, the coupling tubes having the same configuration are provided on the distal end portion 5 side and the flexible tube section 7 side of the bending section 6. However, it is also possible to dispose coupling tubes having different configurations on the distal end portion 5 side and the flexible tube section 7 side of the bending section 6 and set a bent shape of the bending section as appropriate.

As the different configurations of the coupling tubes, the first coupling tube 21 provided on the distal end portion 5 side and the second coupling tube 27 provided on the flexible tube section 7 side are set to different lengths or rigidity in the uncompressed state or rigidity in the compressed state is differentiated in the first coupling tube 21 provided on the distal end portion 5 side and the second coupling tube 27 provided on the flexible tube section 7 side. That is, crude density of the array interval of the joint pieces configuring the coupling tubes is changed or materials of the coupling tubes are differentiated.

It is also possible to provide the switching sections 20 and 20A in the first part of the bending section of the endoscope described in Japanese Patent No. 4856289 and, in the bending section, change rigidity on the distal end side of the first part to low rigidity, high rigidity, or the like.

In the embodiment, the pipe-like member that can be switched to the uncompressed state and the compressed state with respect to the longitudinal axis direction is the coupling tube by the laser-cut pipe. However, a stretchable spiral tube may be provided in the bending section center instead of the laser-cut pipe that can be switched to the uncompressed state and the compressed state.

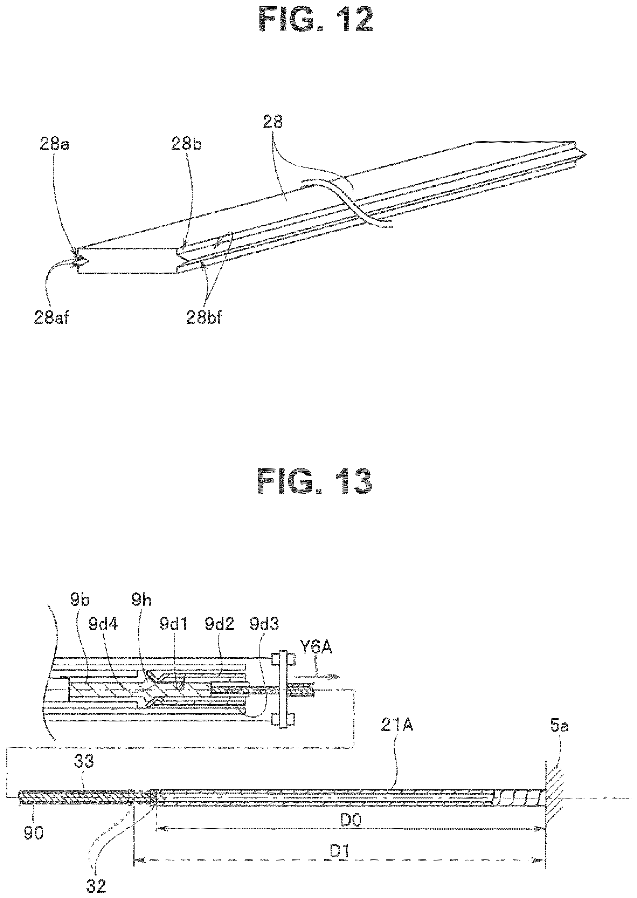

A configuration example in which a stretchable spiral tube is provided in a center of a distal end side bend of a bending section is explained with reference to Fig. 12 and Fig. 13.

When a spiral tube 21A is used, a sectional shape of an element wire forming the spiral tube 21A is suitably a flat shape rather than a circular shape. Further, a flat plate 28 shown in Fig. 12 is optimum. The flat plate 28 includes, on one short side, a V groove 28a having a V shape and includes, on the other short side, a V protrusion 28b formed in V shape and disposed to engage in the V groove 28a.

The spiral tube 21A formed by winding the flat plate 28 in a spiral shape is a densely wound coil sheath. In an extended state, a predetermined gap is provided between a pair of inclined surfaces 28af of the V groove 28a and a pair of inclined surfaces 28bf of the V protrusion 28b. The spiral tube 21A has first rigidity. On the other hand, in a contracted state, the spiral tube 21A changes to a predetermined contact state in which the inclined surfaces 28af of the V groove 28a and the inclined surface 28bf of the V protrusion 28b are in contact. The spiral tube 21A has second rigidity. That is, the spiral tube 21A is a so-called drawn spring and has a predetermined elastic force.

The spiral tube 21A has a natural length in the contracted state. The natural length is the same as D0 in the present embodiment.

In the present embodiment, as shown in Fig. 13, a distal end face of the spiral tube 21A is directly bonded to a proximal end face of the distal end rigid portion 5a or integrally fixed to the proximal end face of the rigid portion 5a via another member or the like. On the other hand, the distal end portion of the operation wire 33 is directly fixed to a proximal end face of the spiral tube 21A or integrally fixed to the proximal end face of the spiral tube 21A after the second stopper 32 or the like is provided.

Therefore, in the present embodiment, the setting hole 5h is unnecessary in the distal end rigid portion 5a. The lid body 5c and the first stopper 31 are also unnecessary.

The other components are the same as the components shown in Fig. 3 explained above.

With the configuration, in a state in which the slide member convex section 9h is held by the locking convex sections 9d4 of the leaf springs 9d2 and 9d3, the disposition position of the distal end face of the second stopper 32 integrally fixed to the operation wire 33 is a position where a distance from the proximal end face of the distal end rigid portion 5a is D1. As a result, the spiral tube 21A is switched to the extended state and changes to a state in which rigidity decreases.

Switching from the extended state to the contracted state of the spiral tube 21A is explained.

When switching the spiral tube 21A from the extended state to the contracted state, the user operates the switching lever 9a to release the restriction of the slide member convex section 9h. Then, the spiral tube 21A contracted to be the densely wound coil sheath in a natural state returns to the contracted state, which is the natural state, with a restoring force of the spiral tube 21A. The distance from the proximal end face of the distal end rigid portion 5a decreases to D0.

As a result, it is possible to obtain action and effects same as the action and the effects in the embodiment explained above.

Note that, when a second spiral tube 21A is provided in the proximal end side bending portion, fixing by adhesion or the like is made unnecessary by adopting a configuration in which a distal end face of the second spiral tube 21A is disposed to be pressed against the proximal end face of the third stopper 35 with an urging force of the spiral tube 21A.

In the embodiment explained above, the coupling tube 21 or the spiral tube 21A is provided in the substantial center portion in the bending section 6. However, the disposition position of the coupling tube for selectively switching the rigidity on the distal end side of the bending section 6 is not limited to the substantial center portion in the bending section 6.

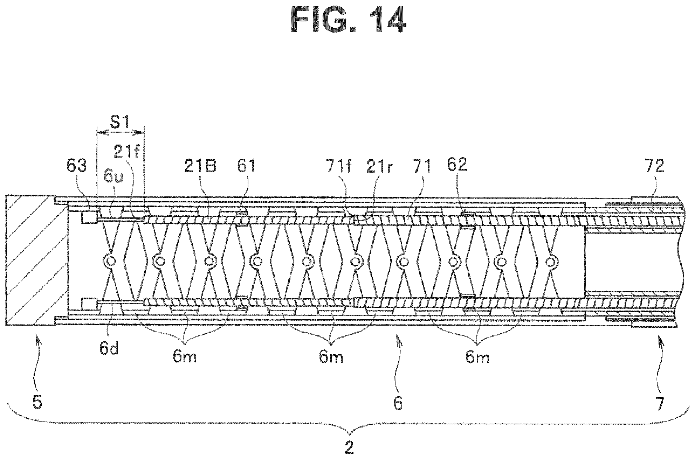

A second embodiment is explained with reference to Fig. 14 to Fig. 16B.

As shown in Fig. 14, in the present embodiment, a spiral tube 21B, rigidity of which is selectively switched, is provided near a bending section inner circumferential surface in the distal end side bending portion of the bending section 6.

The spiral tube 21B is stretchable and is a coil sheath through which the bending wires 6u and 6d are respectively slidably inserted. The spiral tube 21B is disposed to be slidably inserted through one or a plurality of coil receivers 61 fixedly provided to the predetermined bending pieces 6m.

A sectional shape of an element wire forming the spiral tube 21B is the same as the sectional shape of the element wire forming the spiral tube 21A. As shown in Fig. 12, the element wire includes, on one short side, the V groove 28a having a V shape and includes, on the other short side, the V protrusion 28b formed in V shape and disposed to engage in the V groove 28a.

The spiral tube 21B in the present embodiment is a sparsely wound coil sheath formed by winding the flat plate 28 in a spiral shape. In the present embodiment, the spiral tube 21B has a natural length in an extended state and has first rigidity. In a compressed state, the spiral tube 21B changes to a predetermined adhered state in which the inclined surface 28af of the V groove 28a and the inclined surface 28bf of the V protrusion 28b adhere to each other. The spiral tube 21B has second rigidity.

That is, the spiral tube 21B is a so-called compression spring and has a predetermined elastic force.

On the other hand, an inner coil sheath 71 is a densely wound coil of a flat wire.

A distal end of the spiral tube 21B is a free end in the bending section 6. A proximal end face 21r of the spiral tube 21B is integrally fixed to a distal end face 71f of the inner coil sheath 71. A proximal end face 71r of the inner coil sheath 71 is extended into the operation section 3 through an outer coil sheath 72.

Therefore, in the bending section 6, the spiral tube 21B is disposed on the bending section distal end side. A distal end side portion of the inner coil sheath 71 is disposed on the bending section proximal end side.

The inner coil sheath 71 is disposed to be slidably inserted through one or a plurality of coil receivers 62 fixedly provided to the predetermined bending pieces 6m.

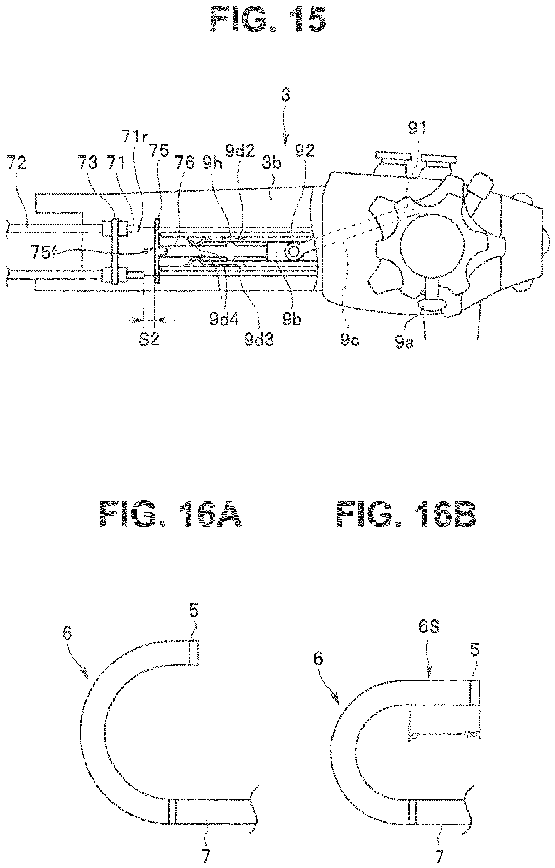

Reference sign S1 denotes a first contact prevention space. The first contact prevention space S1 is a spiral tube movement space provided between a wire stop 62 and a distal end face 21f of the spiral tube 21B. The first contact prevention space S 1 prevents, when the bending section 6 bends, both ends of the spiral tube 21A or 21B from coming into contact with another member to be compressed and unable to further bend. The proximal end face 71r of the inner coil sheath 71 is located further on a proximal end side than a fixed plate 73 fixedly provided to the base plate 3b as shown in Fig. 15 and opposed to a distal end face 75f of a contact plate 75 explained below.

A second contact prevention space S2 having the same purpose as the first contact prevention space S1 is provided between the proximal end face 71r of the inner coil sheath 71 and the distal end face 75f of the contact plate 75.

Therefore, in a state in which the contact prevention spaces S 1 and S2 are secured, the spiral tube 21B and the inner coil sheath 71 are capable of sliding in the outer coil sheath 72. Therefore, the bending section 6 smoothly performs a bending motion and bends in a predetermined bent shape as shown in Fig. 16A.

Note that, in the bending section 6, flexibility of the spiral tube 21B and flexibility of the inner coil sheath 71 are substantially equally set such that the bending section 6 substantially uniformly bends over bending section total length.

As shown in Fig. 15, a distal end portion of the outer coil sheath 72 is fixed to a distal end side inner surface of the flexible tube section 7. A proximal end face of the outer coil sheath 72 is fixed to the fixed plate 73 in the operation section 3.

The contact plate 75 that comes into contact with the proximal end face 71r of the inner coil sheath 71 is provided in the operation section 3. The distal end face 75f of the contact plate 75 is a contact surface in contact with the proximal end face 71r of the inner coil sheath 71 and is a plane. A spherical section 76 is provided in a center of a proximal end face of the contact plate 75. The spherical section 76 is a ball joint coupled to a distal end portion of a slide member 9b. The contact plate 75 provided at a distal end portion of the slide member 9b is capable of swinging centering on the spherical section 76.

Therefore, as shown in Fig. 15, when the flexible tube section 7 loops, even if a position of the proximal end face 71r of the inner coil sheath 71 deviates according to an inner and outer circumference difference of the loop, when the slide member 9b moves in a direction of the insertion section 2, the distal end face 75f of the contact plate 75 comes into contact with the proximal end face 71r of the inner coil sheath 71 stepwise and changes to a predetermined contact state.

With this configuration, in a substantially straight state of the bending section 6, by operating the switching lever 9a provided in the operation section 3, the slide member 9b is moved as explained above. Then, the distal end face 75f of the contact plate 75 comes into contact with the proximal end face 71r of the inner coil sheath 71.

Thereafter, the inner coil sheath 71 inserted through the outer coil sheath 72 is moved to the distal end side according to the movement of the slide member 9b. The spiral tube 21 B is also moved to the distal end side according to the movement of the inner coil sheath 71.

The spiral tube 21 B is further moved to the distal end side according to the movement of the inner coil sheath 71, whereby the distal end face 21 f of the spiral tube 21B comes into contact with the wire stop 63. Thereafter, the spiral tube 21B is compressed by the movement of the inner coil sheath 71. In the spiral tube 21B, the inclined surface 28af of the V groove 28a and the inclined surface 28bf of the V protrusion 28b of the element wire adjacent to each other come into contact with each other and change to the adhered state.

The slide member convex section 9h climbs over the locking convex sections 9d4, whereby the spiral tube 21B is retained in the adhered state.

As a result, the distal end side bending portion of the bending section 6 less easily bends. In this state, by bending the bending section 6, as shown in Fig. 16B, a bent shape including the straight shape section 6S in the distal end side bending portion of the bending section 6 is obtained. It is possible to obtain action and effects same as the action and effects explained above.

Note that, in the adhered state of the element wire of the spiral tube 21B, the distal end face 75f of the contact plate 75 is separated from the proximal end face 71r of the inner coil sheath 71 by performing operation for moving the slide member 9b in a direction opposite to the direction described above. Then, the spiral tube 21B is extended by an urging force of the compressed spiral tube 21B. The inner coil sheath 71 moves to the proximal end side.

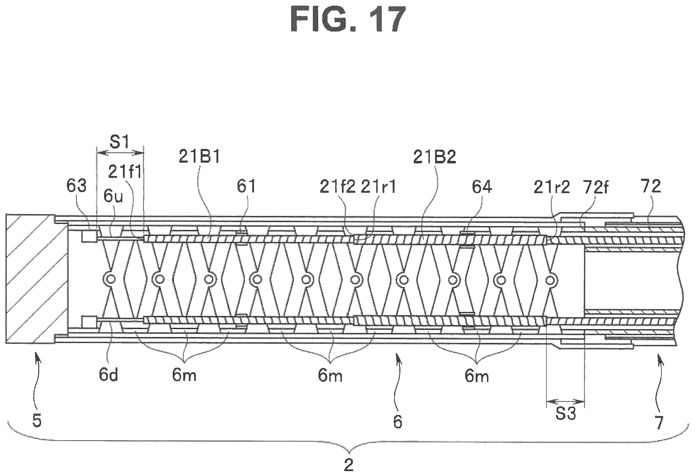

A configuration in which the spiral tubes 21B are provided in the distal end side bending portion and the proximal end side bending portion of the bending section is explained with reference to Fig. 17 to Fig. 19C.

As shown in Fig. 17, in the present embodiment, a first spiral tube 21B1 having a configuration same as the configuration of the spiral tube 21B explained above is provided in the distal end side bending portion of the bending section 6. On the other hand, a second spiral tube 21B2 is provided in the proximal end side bending portion.

As shown in Fig. 14, a distal end of the first spiral tube 21B1 is a free end in the bending section 6. A proximal end face 21r1 of the first spiral tube 21B1 is integrally fixed to the distal end face 71 f of the inner coil sheath 71.

On the other hand, the second spiral tube 21B2 is slidably disposed on an outer circumferential surface side of the inner coil sheath 71. In other words, a distal end side portion of the inner coil sheath 71 is inserted through a through-hole of the second spiral tube 21B2.

The second spiral tube 21B2 is disposed to be slidably inserted through a coil sheath receiver 64 fixedly disposed to the predetermined bending pieces 6m of the bending section 6.

A distal end portion of the second spiral tube 21B2 is integrally fixed to a distal end portion of the inner coil sheath 71 by adhesion or bonding. A proximal end face 21r2 of the second spiral tube 21B2 comes into contact with a distal end face 72f of the outer coil sheath 72. Like the first contact prevention space S1, a third contact prevention space S3 is provided such that the bending section can bend.

The third contact prevention space S3 is a spiral tube movement space provided between the proximal end face 21r2 of the second spiral tube 21B2 and the distal end face 72f of the outer coil sheath 72. In a state in which the contact prevention spaces S1, S2, and S3 are secured, the second spiral tube 21B2 is capable of advancing and retracting in addition to the first spiral tube 21B1 and the inner coil sheath 71.

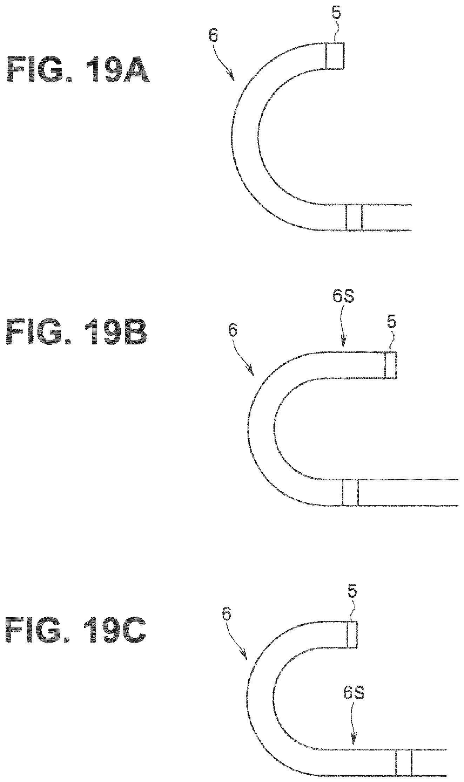

Note that, in the present embodiment, in order to make it possible to smoothly bend the bending section 6 over bending section entire length as shown in Fig. 19A, flexibility of the first spiral tube 21B1 and flexibility of the inner coil sheath 71 and the second spiral tube 21B2 disposed on an outer circumferential surface of the inner coil sheath 71 are set to be substantially equal.

That is, the inner coil sheath 71 is formed to have more flexibility compared with the inner coil sheath 71 explained above. The second spiral tube 21 B2 is also formed to have more flexibility compared with the first spiral tube 21B1.

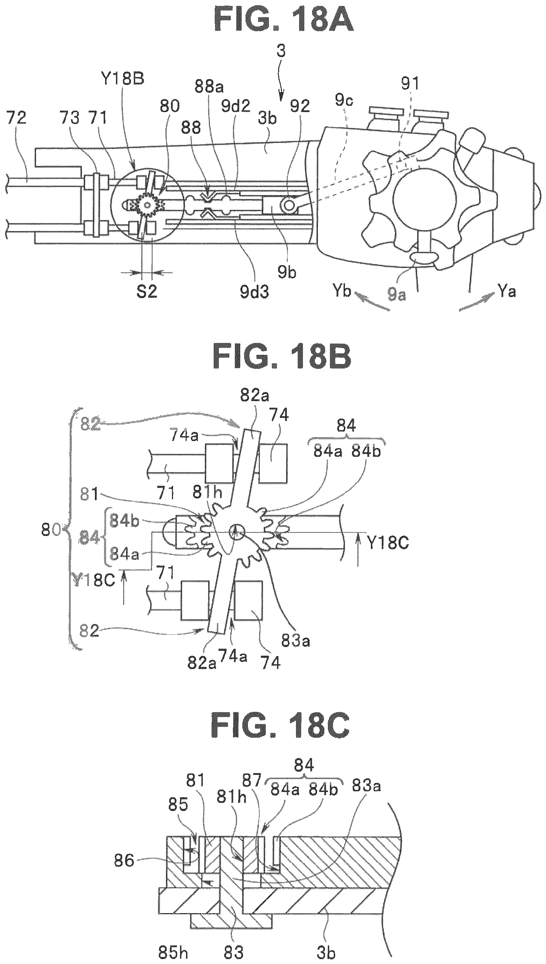

As shown in Fig. 18A to Fig. 18C, an inner coil sheath moving member 80 is provided in the operation section 3. The inner coil sheath moving member 80 includes an attachment section 81 having a substantially columnar shape and a pair of protrusion sections 82 projecting outward from an outer circumferential surface of the attachment section 81.

The attachment section 81 includes a shaft hole 81h in a center thereof. The pair of protrusion sections 82 are provided in a symmetrical positional relation across a center axis of the shaft hole 81h.

The shaft hole 81h of the attachment section 81 is disposed in a shaft section 83a of a shaft member 83 provided to project from the base plate 3b, whereby the inner coil sheath moving member 80 is disposed to be capable of turning around an axis.

An outer teeth section 84a functioning as a meshing section 84 is formed on the outer circumferential surface of the attachment section 81.

The inner coil sheath 71 is extended into the operation section 3 as explained above. In the present embodiment, coil sheath stops 74 are fixed to respective proximal end portions of the inner coil sheath 71. Circumferential grooves 74a are formed in longitudinal direction middles of the coil sheath stops 74. In the circumferential grooves 74a, distal end portions 82a of protrusion sections 82 of the inner coil sheath moving member 80 are disposed.

In the present embodiment, a concave section 85, in which the attachment section 81 is disposed, is provided at the distal end portion of the slide member 9b. An opening of a long hole 85h extending in a longitudinal axial direction, in which the shaft section 83a passes, is formed in a bottom surface of the concave section 85.

The concave section 85 includes a distal end side wall 86 and a proximal end side wall 87. Inner teeth sections 84b functioning as meshing sections 84 meshing with the outer teeth section 84a are formed in the respective walls.

In the present embodiment, the slide member 9b moves to a first position, a second position, and a third position.

When the slide member 9b is located in the first position shown in Figs. 18A, 18B, and 18C, the outer teeth section 84a of the attachment section 81 and the inner teeth sections 84b provided in the distal end side wall 86 and the proximal end side wall 87 of the concave section 85 are in a non-meshed state.

By advancing the slide member 9b from this state, the inner teeth section 84b provided in the proximal end side wall 87 of the concave section 85 meshes with the outer teeth section 84a of the attachment section 81. On the other hand, by retracting the slide member 9b, the inner teeth section 84b provided in the distal end side wall 86 of the concave section 85 meshes with the outer teeth section 84a of the attachment section 81.

In the present embodiment, when positions of the coil sheath stops 74 of the inner coil sheath 71 deviate according to an inner and outer circumference difference in a loop when the flexible tube section 7 loops, the inner teeth section 84b and the outer teeth section 84a mesh with each other, whereby the inner coil sheath 71 can be moved to the distal end side or the proximal end side in a state in which the positional deviation of the coil sheath stops 74 is maintained.

Note that reference numeral 88 denotes a circumferential groove having a V shape. Reference sign 88a denotes a first circumferential convex section and reference sign 88b denotes a second circumferential convex section.

With this configuration, in a substantially straight state of the bending section 6, the switching lever 9a provided in the operation section 3 is operated in an arrow Ya direction in Fig. 18A, whereby the slide member 9b moves toward the insertion section 2.

Then, the locking convex sections 9d4 come off the circumferential groove 88. The proximal end side wall 87 of the concave section 85 advances toward the attachment section 81. The inner teeth section 84b of the proximal end side wall 87 meshes with the outer teeth section 84a of the attachment section 81. Then, the inner coil sheath moving member 80 starts advance while maintaining the meshed state.

The distal end portions 82a of the protrusion sections 82 of the inner coil sheath moving member 80 come into contact with distal end side walls of the circumferential grooves 74a, whereby the inner coil sheath 71 is moved to the distal end side. The first spiral tube 21B1 and the second spiral tube 21B2 integral with the inner coil sheath 71 also move to the distal end side according to the movement of the inner coil sheath 71.

The first spiral tube 21B1 comes into contact with the wire stop 63 as explained above. Thereafter, the first spiral tube 21B1 is compressed by the inner coil sheath 71 and changes to the adhered state. The first circumferential convex section 88a passes the locking convex section 9d4, whereby the first spiral tube 21B1 is retained in the adhered state. At this point, the second spiral tube 21B2 remains in the sparsely wound coil sheath state.

As a result, in the bending section 6, the distal end side bending portion less easily bends. In this state, by bending the bending section 6, as shown in Fig. 19B, a bent shape including the straight shape section 6S in the distal end side bending portion of the bending section 6 is obtained. It is possible to obtain action and effects same as the action and the effects explained above.

Note that, in the adhered state of the element wire of the first spiral tube 21B1, operation for moving the slide member 9b in an arrow Yb direction, which is a direction opposite to the direction explained above, is performed, whereby the first circumferential convex section 88a passes the locking convex sections 9d4 and the slide member 9b starts retraction.

The distal end portions 82a of the protrusion sections 82 come into contact with the proximal end side walls of the circumferential grooves 74a, whereby the inner coil sheath 71, the second spiral tube 21B2, and the first spiral tube 21B1 are moved to the operation section 3 side.

At this point, the compressed first spiral tube 21B1 returns to the extended state with a restoring force. Thereafter, the first spiral tube 21B1 is moved according to the movement of the inner coil sheath 71. The first contact prevention space S1 is set again.

On the other hand, in the substantially straight state of the bending section 6, the switching lever 9a provided in the operation section 3 is operated in an arrow Yb direction in Fig. 18A, whereby the slide member 9b moves toward the lever 9a direction.

Then, the locking convex sections 9d4 come off the circumferential groove 88 and the distal end side wall 86 of the concave section 85 retracts toward the attachment section 81. The inner teeth section 84b of the distal end side wall 86 meshes with the outer teeth section 84a of the attachment section 81. Then, the inner coil sheath moving member 80 starts retraction while maintaining the meshed state.

The distal end portions 82a of the protrusion sections 82 of the inner coil sheath moving member 80 come into contact with the proximal end side walls of the circumferential grooves 74a, whereby the inner coil sheath 71 is moved to the proximal end side. The first spiral tube 21B1 and the second spiral tube 21B2 integral with the inner coil sheath 71 also move to the proximal end side according to the movement of the inner coil sheath 71.

A proximal end face of the second spiral tube 21B2 comes into contact with the distal end face 72f of the outer coil sheath 72. Thereafter, the inner coil sheath 71 is further moved to the proximal end side, whereby the second spiral tube 21B is compressed by the inner coil sheath 71 and changes to the adhered state.

The second circumferential convex section 88b passes the locking convex sections 9d4, whereby the second spiral tube 21B2 is retained in the adhered state. At this point, the first spiral tube 21B1 remains in the sparsely wound coil sheath state.

As a result, in the bending section 6, the proximal end side bending portion less easily bends. In this state, by bending the bending section 6, a bent shape including the straight shape section 6S in the proximal end side bending portion of the bending section 6 as shown in Fig. 19C may be obtained.

Note that, in the adhered state of the element wire of the second spiral tube 21B2, operation for moving the slide member 9b in the arrow Ya direction, which is a direction opposite to the direction explained above, is performed, whereby the second circumferential convex section 88b passes the locking convex sections 9d4 and the slide member 9b starts advance.

The distal end portions 82a of the protrusion sections 82 come into contact with the distal end side walls of the circumferential grooves 74a, whereby the inner coil sheath 71, the second spiral tube 21B2, and the first spiral tube 21B1 are moved to the distal end portion 5 side.

At this point, the compressed second spiral tube 21B2 returns to the extended state with a restoring force. Thereafter, the second spiral tube 21B2 is moved according to the movement of the inner coil sheath 71. The third contact prevention space S3 is set again.

Note that, instead of the contact plate 75 shown in Fig. 15, it is also possible to provide the inner coil sheath moving member 80 and fixedly provide the coil sheath stops 74 to the respective proximal end portions of the inner coil sheath 71. As a result, it is possible to surely return the spiral tube 21B to a position where the first contact prevention space S1 is set again.

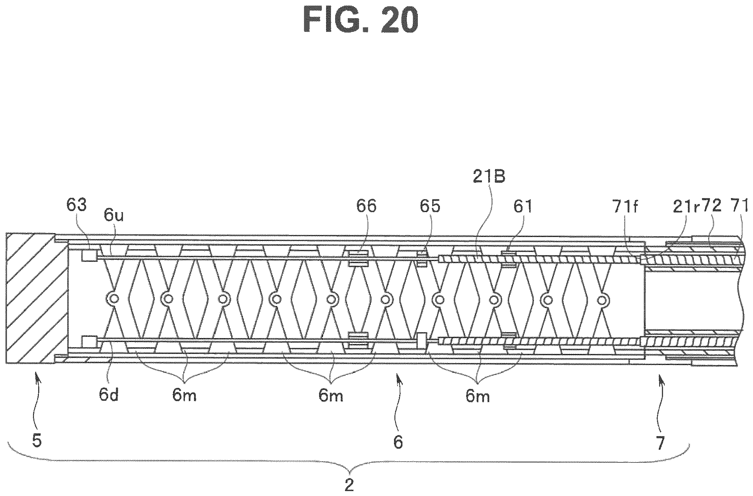

It is also possible to provide the spiral tube 21 B in the proximal end side bending portion of the bending section 6 as shown in Fig. 20 and provide a spiral tube receiver 65 in the predetermined bending pieces 6m of the bending section 6. The distal end face 21f of the spiral tube 21B comes into contact with a proximal end face 65r of the spiral tube receiver 65.

A through-hole through which the bending wires 6u and 6d are slidably inserted is formed in the spiral tube receiver 65. Reference numeral 66 denotes a bending wire receiver. The bending wires 6u and 6d are disposed to be inserted through the bending wire receiver.

With this configuration, it is possible to move the inner coil sheath 71, bring the distal end face 21f of the spiral tube 21B into contact with the spiral tube receiver 65, and compress the spiral tube 21B. As a result, a bent shape including the straight shape section 6S in the proximal end side bending portion of the bending section 6 as shown in Fig. 19C can be obtained.

According to the present invention, it is possible to realize an endoscope in which rigidity of a distal end side of a bending section can be selectively changed according to an observation or a manipulation.

The present invention is not limited only to the embodiments explained above and can be variously modified and carried out in a range not departing from the spirit of the invention.

This application is filed claiming priority from Japanese Patent Application No. 2015-144144 filed in Japan on July 21, 2015, disclosed contents of which are incorporated in this specification and the claims by reference.

## Claims

1. An endoscope comprising:
a bending section provided in an insertion section inserted into a subject, the bending section being bendable; and
a switching section capable of changing rigidity on a distal end side of the bending section.

2. The endoscope according to claim 1, wherein
the bending section includes:
a distal end side bending portion forming the distal end side of the bending section; and
a proximal end side bending portion forming a proximal end side of the bending section, and
the switching section is capable of changing rigidity of the distal end side bending portion.

3. The endoscope according to claim 2, wherein
the switching section includes:
a coupling tube disposed on an inside of a plurality of bending pieces of the bending section and configured in a tubular shape by concatenating a first annular section and a second annular section to be capable of coming into contact with each other, the coupling tube being capable of changing a separation distance between an inclined surface of the first annular section and an inclined surface of the second annular section adjacent to each other; and
a coupling tube operation section that reduces the separation distance and disposes the inclined surface of the second annular section to be superimposed on the inclined surface of the first annular section in an insertion axial direction of the insertion section.

4. The endoscope according to claim 3, wherein the coupling tube is stretchable according to operation of the coupling tube operation section.

5. The endoscope according to claim 3, further comprising:
a wire inserted through an inside of the coupling tube;
a first restricting section fixed to a distal end of the wire; and
a second restricting section fixed to a halfway portion of the wire, the second restricting section restricting movement of an end portion on a proximal end side of the coupling tube with respect to the wire.

6. The endoscope according to claim 5, further comprising a compression spring having an urging force for disposing the first restricting section in a predetermined position on a distal end side of the insertion section.

7. The endoscope according to claim 3, further comprising:
a first coupling tube disposed in the distal end side bending portion; and
a second coupling tube disposed in the proximal end side bending portion.

8. The endoscope according to claim 3, further comprising a bending wire towed in order to bend the bending section, wherein
the bending wire is disposed to be inserted through an inside of a coil sheath that is stretchable and has a natural length in an extended state.

9. The endoscope according to claim 8, further comprising:
an inner coil sheath having flexibility, a distal end side, through which the bending wire is inserted, of the inner coil sheath being provided in the bending section and a proximal end side of the inner coil sheath being provided in an operation section; and
an outer coil sheath in which the inner coil sheath is slidably disposed, a distal end portion of the inner coil sheath being led out from a distal end face of the outer coil sheath and a proximal end portion of the inner coil sheath being led out from a proximal end face of the outer coil sheath, wherein
the inner coil sheath moves in the outer coil sheath according to operation of the coupling tube operation section and brings element wires adjacent to each other of the coil sheath into an adhered state.
